# EUROPÄISCHE PATENTSCHRIFT

(11) **EP 2 249 710 B1**
(45) Veröffentlichungstag und Bekanntmachung des Hinweises auf die Patenterteilung: **20.04.2016**
(21) Anmeldenummer: 09718151.5
(22) Anmeldetag: 02.03.2009
(51) Int. Cl.: A61B 17/04, A61F 2/00, A61B 17/00

(54) **VORRICHTUNG ZUR VERANKERUNG EINES FADENS IN GEWEBE**
DEVICE FOR ANCHORING A SUTURE IN TISSUE
DISPOSITIF POUR L'ANCRAGE D'UN FIL DANS UN TISSU

(30) Priorität: 03.03.2008 US 33066 P
(43) Veröffentlichungstag der Anmeldung: 17.11.2010
(73) Patentinhaber: Woodwelding AG, 6304 Zug (CH)
(72) Erfinder: LEHMANN, Mario, 2353 Les Pommerats (CH); TORRIANI, Laurent, 2516 Lamboing (CH); MEHL, Stephanie, 8932 Mettmenstetten (CH); MAYER, Jörg, 5702 Niederlenz (CH)
(74) Vertreter: Frei Patent Attorneys
(86) Internationale Anmeldenummer: PCT/CH2009/000082
(87) Internationale Veröffentlichungsnummer: WO 2009/109057

(56) Entgegenhaltungen:
- US-A1- 2007 265 704
- US-A1- 2007 270 974

## Beschreibung

Die Erfindung liegt auf dem Gebiete der Medizinaltechnik und betrifft eine Vorrichtung zur Verankerung eines Fadens in Gewebe, insbesondere in Knochengewebe.

In Knochengewebe verankerte Fäden werden in der Human- und Veterinärmedizin beispielsweise verwendet für die Wieder-Befestigung von Sehnen und Bändern an Knochen im Bereich von Gelenken. Der Knochen, in dem solche Verankerungen vorgenommen werden, weist eine äussere, dichte Knochenschicht (Kortikalis) mit einer Dicke in der Grössenordnung von 1 bis 2 mm auf, die gegen innen in weniger dichten Knochen (Spongiosa) übergeht. Je nach Patient hat der spongiöse Knochen sehr verschiedene Eigenschaften, insbesondere kann er je nach Alter und Gesundheitszustand des Patienten eine sehr unterschiedliche mechanische Stabilität und Regenerationsfähigkeit aufweisen.

Gemäss dem Stande der Technik werden für die Verankerungen von Fäden in Knochengewebe beispielsweise schraubenförmige Anker verwendet, die beispielsweise in eine in der Kortikalis vorgebohrte Öffnung eingeschraubt werden und deren Rückhalt im Gewebe insbesondere durch die Kortikalis gewährleistet werden muss, wenn die Spongiosa eine nur kleine mechanische Stabilität aufweist. Auch radial komprimierbare Anker werden für die Verankerung von Fäden in Knochengewebe verwendet. Solche Anker sind beispielsweise mit deformierbaren Widerhaken ausgerüstet und werden bis in die Spongiosa eingeführt, wo sie je nach mechanischem Widerstand expandieren und/oder sich verhaken. Auch diese Anker werden beispielsweise in eine mindestens in der Kortikalis vorgebohrten Öffnung eingeschlagen. Eine dritte bekannte Art von Fadenankern wird durch eine mindestens in der Kortikalis vorgebohrte Öffnung eingebracht bis unter die Kortikalis, um dann im spongiösen Knochen meist mit Hilfe des Fadens um ca. 90° gedreht zu werden. Durch die Drehung wird der Anker, der länger ist als breit, in eine Lage gebracht, in der er nicht mehr durch die Öffnung entfernt werden kann und dadurch unter der Kortikalis verankert ist. In den Publikationen WO 02/069817, WO 04/017857 und WO 05/079696 sind auch für Fadenanker geeignete Verankerungen offenbart, die auf einer Verflüssigung eines thermoplastischen Materials mittels mechanischer Schwingungen, beispielsweise Ultraschallschwingungen beruhen, wobei für die Verflüssigung eine Reibung zwischen dem Knochengewebe und dem thermoplastischen Material notwendig ist, die eine minimale mechanische Festigkeit dieses Materials voraussetzt.

Eine Vorrichtung gemäß der Präambel des Hauptanspruchs 1 ist in der Druckschrift US-A-2007/0265704 offenbart.

Zur Halterung des Fadens weisen die bekannten Fadenanker beispielsweise proximale Ösen auf, wobei Anker und Ösen insbesondere derart ausgebildet sind, dass der implantierte Anker nicht über die Knochenoberfläche vorsteht und der Faden auch nach der Implantation des Ankers frei durch die Öse gleiten kann. Damit der verankerte Faden, wenn er zur Befestigung der Sehne oder des Bandes gespannt wird, möglichst wenig in das Knochengewebe einschneidet, ist es vorteilhaft, dass er möglichst nahe an der Knochenoberfläche am Anker befestigt ist. Von den Fadenankern wird ferner gefordert, dass die Festigkeit der Verankerung und der Öse etwa der Fadenfestigkeit entspricht. Nicht nur für Anwendungen in minimal invasiven Operationen sondern auch, damit für die Anker im Knochengewebe möglichst kleine Bohrungen gemacht werden müssen und die Anker möglichst nahe beieinander gesetzt werden können, sind kleine Ankerquerschnitte in der Grössenordnung von 5 mm oder weniger erwünscht. Ebenso ist es wünschenswert, dass die Verankerung vom Chirurgen mit nur einer Hand durchgeführt werden kann.

Die oben genannten, bekannten Fadenanker sind üblicherweise je für spezifische Verankerungsstellen und meist auch je für spezifische Knochenqualitäten konzipiert, dies nicht zuletzt deshalb, weil die genannten Verankerungsprinzipien nicht sehr allgemein anwendbar sind. Das heisst mit anderen Worten, dass der Chirurg je nach Operation den einen oder anderen Ankertyp auszuwählen hat oder sogar für eine einzige Operation verschiedene Ankertypen verwenden muss.
Die Erfindung stellt sich die Aufgabe, eine Vorrichtung zum Verankern eines Fadens in Gewebe, insbesondere in Knochengewebe, zu schaffen, wobei die Vorrichtung den oben genannten Bedingungen Rechnung tragen und die genannten Wünsche erfüllen soll. Ferner soll die Verankerung von der mechanischen Qualität des Gewebes weitgehend unabhängig oder ad hoc auf eine vorliegende solche Qualität abstimmbar sein.
Diese Aufgabe wird gelöst durch die Vorrichtung zur Verankerung eines Fadens in Gewebe, wie sie in den Patentansprüchen definiert ist.
Die erfindungsgemässe Vorrichtung zur Verankerung eines Fadens in Gewebe basiert auf der Verankerungsmethode, die in der Druckschrift WO-A-2009/055952 beschrieben ist. Diese Druckschrift ist ein Dokument gemäß Artikel 54(3) EPÜ.
Die genannte Verankerungsmethode beruht darauf, zwischen zwei Ankerteilen ein durch mechanische Schwingungen verflüssigbares Material zu verflüssigen, dadurch, dass die beiden Ankerteile aneinander gehalten werden und der eine der Ankerteile durch eine direkte oder indirekte Kopplung an eine entsprechende Schwingungsquelle (z.B. Ultraschallvorrichtung) zu mechanischen Schwingungen (z.B. Ultraschallschwingungen) angeregt wird. Dabei werden die beiden Ankerteile gegeneinander bewegt, so dass das verflüssigte Material von zwischen den beiden Teilen ausfliesst und benachbartes Gewebe interpenetriert, was nach der Wiedererstarrung des verflüssigten Materials zu einem Formschluss zwischen Anker und Gewebe und vorteilhafterweise auch zwischen den beiden Ankerteilen führt.

Für den Verankerungsprozess werden die Ankerteile in einer Gewebeöffnung positioniert, wobei die Öffnung einen leicht grösseren Querschnitt aufweist als die Ankerteile und diese somit im wesentlichen ohne Belastung des Gewebes positioniert werden können. Die einzige Interaktion mit dem Gewebe ist die genannte Interpenetration durch das verflüssigte Material, die aber auch in mechanisch wenig widerstandsfähigem Gewebe möglich ist und solches Gewebe noch verstärken kann. Die Menge an verflüssigtem Material kann durch einen entsprechenden Hub der Ankerteile gegeneinander für jede spezifische Verankerung ad hoc in einfachster Weise eingestellt oder vom Chirurgen sogar während des Verankerungsvorgangs entsprechend gewählt werden. Aus den genannten Gründen ist das Verankerungsprinzip, auf dem die erfindungsgemässe Vorrichtung basiert, nicht nur für Knochengewebe mit verschiedensten mechanischen Eigenschaften sondern auch für andere Gewebe geeignet und aus diesem Grund genereller anwendbar als andere bekannte Verankerungsmethoden.

Die erfindungsgemässe Vorrichtung weist einen Anker mit einem Durchgang (z.B. Öse) und einen Faden auf, wobei der Faden frei durch den Durchgang laufend angeordnet ist. Die erfindungsgemässe Vorrichtung weist ferner eine Sonotrode (Schwingungsübertragungselement), eine Führungshülse und gegebenenfalls weitere Elemente auf. Die Bestandteile der Vorrichtung sind relativ zueinander im wesentlichen gebrauchsfertig angeordnet.

Der Anker bildet das distale Ende der Vorrichtung und weist einen distalen Ankerfuss und eine auf dem Ankerfuss aufstehende Verankerungshülse auf, wobei die Verankerungshülse das durch mechanische Schwingungen (z.B. Ultraschall) verflüssigbare Material aufweist. Die Sonotrode erstreckt sich durch die Führungshülse und reicht vom proximalen Ende der Vorrichtung bis an den proximalen Bereich des Ankers. Sie ist ausgerüstet, um im proximalen Bereich an eine Schwingungsquelle angekoppelt zu werden und um die Schwingungen der Schwingungsquelle möglichst verlustfrei an ihr distales Ende zu übertragen. Die Verankerungshülse ist zwischen dem Ankerfuss und der Sonotrode oder einer Stosshülse oder der Führungshülse eingespannt.

Für die Verankerung wird das distale Ende der an die Schwingungsquelle angekoppelten Vorrichtung bzw. der Anker in einer Gewebeöffnung positioniert und die Schwingungsquelle wird aktiviert. Das verflüssigbare Material der eingespannten Verankerungshülse wird verflüssigt durch die Schwingungen oder Vibrationen, die in direktem Kontakt mit der Sonotrode oder über den Ankerfuss auf die Verankerungshülse wirken. Dabei wird die Verankerungshülse mit Hilfe der Sonotrode und anderen Vorrichtungsbestandteilen relativ zum Ankerfuss und gegen diesen bewegt, so dass das verflüssigte Hülsenmaterial unter achsialer Verkürzung der Verankerungshülse radial ausfliesst und das den Anker umgebende Gewebe interpenetriert.

Der Verankerungsprozess kann beispielsweise über die Zeit, in der die Schwingungsquelle eingeschaltet ist, oder über den Hub der Ankerteile relativ zueinander bzw. über die Verkürzung der Verankerungshülse kontrolliert werden und wird durch gegebenenfalls automatisches Abschalten der Schwingungsquelle beendet. Nach dem Wiedererstarren des verflüssigten Materials, was eine nur sehr kurze Zeit (höchstens einige wenige Sekunden) dauert, werden die Sonotrode und das Hülsensystem vom nun verankerten Anker (Ankerfuss und Verankerungshülse) und vom Faden getrennt und die Sonotrode wird von der Schwingungsquelle entkoppelt. Sonotrode und Hülsensystem sind vorteilhafterweise Wegwerfartikel; für das Setzen eines weiteren Ankers wird eine neue Vorrichtung an die Schwingungsquelle gekoppelt.

Es zeigt sich, dass es technisch möglich ist, die Vorrichtung (ausser ganz proximaler Bereich) mit einem Aussendurchmesser des Hülsensystems von weniger als 8 mm und einer Sonotrodenlänge von ca. 10 bis 20 cm zu realisieren, derart, dass sie mit einer in der Arthroskopie üblichen Kanüle verwendbar ist. Die entsprechenden Anker haben Durchmesser von 2 bis 6 mm, insbesondere 3 bis 6 mm und sind für die Verankerung von 1 bis 4 Fäden ausgerüstet. Anker für zwei Fäden haben vorzugsweise einen Durchmesser von ca. 4 mm und axiale Längen von 5 bis 50 mm (vorzugsweise 10 bis 20 mm für Anker mit einem Durchmesser von 4 mm) und sie weisen beispielsweise Durchgänge für zwei Fäden (z.B. zwei Ösen) auf. Für den Anker zu erstellende Öffnungen im Gewebe haben einen Durchmesser, der etwas grösser ist als der Durchmesser des Ankers, vorteilhafterweise beispielsweise um etwa 0,2 mm grösser.

Kreisrunde Querschnitte von Anker und Hülsensystem sind insbesondere aus herstellungstechnischen Gründen bevorzugt. Da aber der Verankerungsprozess im wesentlichen ohne Drehbewegungen auskommt, sind auch andere als kreisrunde Querschnitte möglich.

Die Materialien für Ankerfuss und Verankerungshülse können je nach Anwendung resorbierbar oder nicht resorbierbar sein. Das Material für die Verankerungshülse muss durch mechanische Schwingungen, insbesondere durch Ultraschallschwingungen verflüssigbar sein, mindestens an Oberflächen zwischen einem schwingenden Element und einem gegen das schwingende Element gehaltenen Gegenelement. Solche Materialien sind insbesondere Materialien mit thermoplastischen Eigenschaften. Bevorzugte Materialien für die Verankerungshülse sind resorbierbare Polymere auf der Basis von Milchsäure und/oder Glycolsäure, insbesondere PLLA, PCLA oder PCLLA, insbesondere Poly-LDL-lactid (z.B. erhältlich von Böhringer unter dem Handelsnamen Resomer LR708) oder Poly-DL-lactid (z.B. erhältlich von Böhringer unter dem Handelsnamen Resomer R208). Das Material des Ankerfusses muss mindestens im Bereich der Fadendurchgänge (Ösen) eine mechanische Festigkeit aufweisen, die ein Ausreissen des Fadens zu verhindern mag. Der Ankerfuss besteht aus diesem Grunde beispielsweise aus einem Metall, einem keramischen Material oder einem entsprechend festen Polymer, das ebenfalls ein Thermoplast sein kann. Vorzugsweise besteht der Ankerfuss aus PEEK. Bei entsprechender Dimensionierung kann der Anker auch vollständig (Verankerungshülse und Ankerfuss) aus einem resorbierbarem Polymer bestehen.

Weitere Materialien für die Verankerungshülse sind beispielswseise: nicht resorbierbare Polymere wie Polyolefine (z.B. Polyethylen), Polyacrylate, Polymethacrylate, Polycarbonate, Polyamide (insbesondere Polyamide 11 oder Polyamid 12), Polyester, Polyurethane, Polysulfone, Flüssigkristall-Polymere (LCPs), Polyacetale, halogenierte Polymere, insbesondere halogenierte Polyolefine, Polyphenylene Sulphone, Polysulfone, Polyaryletherketone (z.B. Polyetheretherketon PEEK, erhältlich unter dem Handelsnamen Victrex 450G oder Peek Optima von Invibo), Polyether oder entsprechende Copolymere, Mischpolymere oder Kompositmaterialien, die die genannten Polymere enthalten, oder resorbierbare Polymere wie Polyhydroxyalkanoate (PHA), Polycaprolactone (PCL), Polysaccharide, Polydioxanone (PD), Polyanhydride, Polypeptide, Trimethyl-carbonate (TMC) oder entsprechende Copolymere, Mischpolymere oder Kompositmaterialien, die die genannten Polymere enthalten. Beispielhafte Kompositmaterialien enthalten mindestens ein resorbierbares oder nicht resorbierbares Polymer und Kalziumphosphat (z.B. Hydroxylapatit) als Füllstoff (Füllgehalte vorzugsweise zwischen 10 und 50 Masseprozent).

Anhand der folgenden Figuren werden drei beispielhafte Ausführungsformen der erfindungsgemässen Vorrichtung und deren Funktionsprinzip im Detail beschrieben und von diesen abgeleitete weitere Ausführungsformen erwähnt. Dabei zeigen:
- **Figuren 1 und 2**: eine erste beispielhafte Ausführungsform der erfindungsgemässen Vorrichtung als Ansicht (Fig. 1) und axial geschnitten (Fig. 2);
- **Figuren 3 und 4**: in einem grösseren Massstab den Anker der Vorrichtung gemäss Fig. 1 und 2 (axiale Schnitte mit senkrecht aufeinander stehenden Schnittebenen);
- **Figur 5**: aufeinanderfolgende Phasen des Verankerungsprozesses mit Hilfe der Vorrichtung gemäss Figuren 1 bis 4;
- **Figuren 6 und 7**: eine zweite beispielhafte Ausführungsform der erfindungsgemässen Vorrichtung;
- **Figur 8 und 9**: in einem grösseren Massstab den Anker der Vorrichtung gemäss Fig. 6 und 7 (axialer Schnitt und distale Draufsicht);
- **Figur 10**: aufeinanderfolgende Phasen des Verankerungsprozesses mit Hilfe der Vorrichtung gemäss Figuren 6 bis 9;
- **Figur 11**: eine dritte beispielhafte Ausführungsform der erfindungsgemässen Vorrichtung, axial geschnitten;
- **Figur 12**: aufeinanderfolgende Phasen des Verankerungsprozesses mit Hilfe der Vorrichtung gemäss Fig. 11.
- **Figur 13**: einen weitere beispielhafte Ausführungsform eines Ankers, der für die Vorrichtung gemäss Figur 11 und eine subkortikale Verankerung geeignet ist.

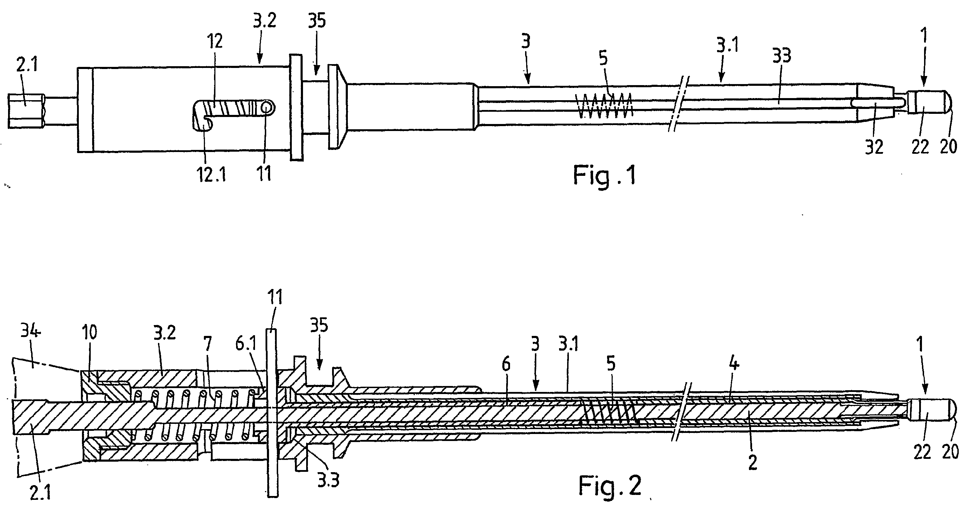

**Figuren 1 bis 4** zeigen eine erste, beispielhafte Ausführungsform der erfindungsgemässen Vorrichtung als Ansicht (Fig.1) und axial geschnitten (Fig. 2) sowie, in einem grösseren Massstab, ein Beispiel eines für diese Ausführungsform geeigneten Ankers in zwei axialen Schnitten (Schnittebenen senkrecht aufeinander stehend).

Die Vorrichtung weist ein proximales Ende (in Fig. 1 und 2 links) und ein dem proximalen Ende gegenüberliegendes, distales Ende (in Fig. 1 und 2 rechts) auf. Der Anker 1 ist am distalen Ende der Vorrichtung angeordnet, eine Sonotrode 2 erstreckt sich axial durch das Lumen einer Führungshülse 3 vom Anker 1 bis an das proximale Ende der Vorrichtung. Das proximale Ende 2.1 der Sonotrode ist ausgerüstet für eine Kopplung an die Schwingungsquelle (nicht dargestellt), beispielsweise mit einer stirnseitigen Vertiefung mit Innengewinde (nicht dargestellt), die an einen entsprechenden, an der Schwingungsquelle angeordneten Gewindezapfen angepasst sind. Zwischen Führungshülse 3 und Sonotrode 2 erstreckt sich eine Stosseinrichtung, die eine distale Stosshülse 4, eine an das proximale Ende der Stosshülse anschliessende Stossfeder 5, eine an das proximale Ende der Stossfeder anschliessende Zwischenhülse 6 und eine an das proximale Ende der Zwischenhülse anschliessende Spannfeder 7 umfasst.

Die Führungshülse 3 weist einen distalen Teil 3.1 mit einem kleineren Durchmesser, beispielsweise ca. 8 mm oder weniger, und einen proximalen Teil 3.2 mit einem grösseren Durchmesser auf. Der distale Teil 3.1 der Führungshülse reicht in den proximalen Teil 3.2 hinein. In diesem Überlappungsbereich sind die beiden Hülsenteile 3.1 und 3.2 beispielsweise miteinander verschraubt, derart, dass die Führungshülse 3 durch weiteres Einschrauben des distalen Teils in den proximalen Teil in einem beschränkten Rahmen verkürzt und durch entsprechendes Ausschrauben verlängert werden kann, wodurch, wie weiter unten erläutert wird, die Verankerungstiefe eingestellt werden kann. Die beiden Führungshülsenteile 3.1 und 3.2 können aber auch fest miteinander verbunden sein. Die genannte Einstellbarkeit wird dann realisiert beispielsweise durch ein in seiner axialen Länge veränderbares Distanzstück 34, das am Gehäuse der Schwingungsquelle beispielsweise ausschraubbar angeordnet ist und an dem sich die Führungshülse bei der Verankerung abstützt.

Der proximale Teil 3.2 der Führungshülse 3 ist durch ein aufgeschraubtes oder aufgeschnapptes Stopfenelement 10 abgeschlossen, wobei das Stopfenelement 10 eine Austrittsöffnung für das proximale Sonotrodenende 2.1 aufweist. Das proximale Sonotrodenende hat einen nicht runden Querschnitt, der dem Querschnitt der Austrittsöffnung im Stopfenelement 10 entspricht, derart, dass das proximale Sonotrodenende 2.1 sich in der Austrittsöffnung nicht drehen kann. Die beiden Querschnitte sind beispielsweise etwa gleich gross und sechseckig. Wenn das proximale Sonotrodenende in der Austrittsöffnung des Stopfenelements 10 positioniert ist, dreht die Sonotrode 2 mit, wenn die Führungshülse 3 gedreht wird, das heisst, kann die Sonotrode durch Drehung der Führungshülse gedreht werden. Wenn die Sonotrode 2 relativ zur Führungshülse 3 weiter proximal positioniert ist, das proximale Sonotrodenende 2.1 also aus dem Stopfenelement 10 ragt, ist die Sonotrode 2 relativ zur Führungshülse 3 frei drehbar.

Im Innenraum des proximalen Teils 3.2 der Führungshülse 3 ist die Spannfeder 7 angeordnet, die zwischen dem Stopfenelement 10 und einem Kopf 6.1 der Zwischenhülse 6 vorgespannt ist, derart, dass dieser Kopf gegen eine innere Schulter 3.3 des proximalen Führungshülsenteils 3.2 gestossen wird. Durch den Kopf 6.1 der Zwischenhülse 6 hindurch verläuft in radialer Richtung ein Entspannungshebel 11 (oder entsprechende Hebelteile sind an diesem Kopf angeformt), der durch beidseitig am proximalen Teil 3.2 der Führungshülse angebrachte, axial verlaufende, schlitzförmige Öffnungen 12 ragt. Die Öffnungen 12 weisen an ihrem proximalen Ende eine um den Umfang des Führungshülsenteils 3.2 verlaufende Verlängerung 12.1 auf. Der Entspannungshebel 11 weist einen Durchgang für die Sonotrode 2 auf, so dass diese in axialer Richtung frei durch den Entspannungshebel 11 bewegbar ist. Der Entspannungshebel 11 wird zusammen mit dem Kopf 6.1 (und damit der ganzen Zwischenhülse 6) durch die Federkraft der vorgespannten Spannfeder 7 gegen das distale Vorrichtungsende gedrückt, so dass der Entspannungshebel im Bereich des distalen Endes der Öffnung 12 positioniert ist. Durch Bewegung des Entspannungshebels 11 in proximaler Richtung und Einrasten in die Verlängerungen 12.1 der Öffnungen 12 wird die Zwischenhülse 6 gegen die Federkraft der Spannfeder 7 in proximaler Richtung verschoben. Durch dieses Spannen der Spannfeder 7 wird die Stossfeder entlastet und damit der über die Stosshülse 4 auf die Verankerungshülse 22 ausgeübte Druck abgebaut. Dadurch wird es möglich, das aus Schwingungsquelle und erfindungsgemässer Vorrichtung bestehende, akustische System bei ungehinderter Schwingfähigkeit abzustimmen und zu testen, ohne dass dabei die Verankerungshülse angeschmolzen wird. Des weiteren wird durch das Spannen der Spannfeder 7 die Montage des Ankers 1 erleichtert.

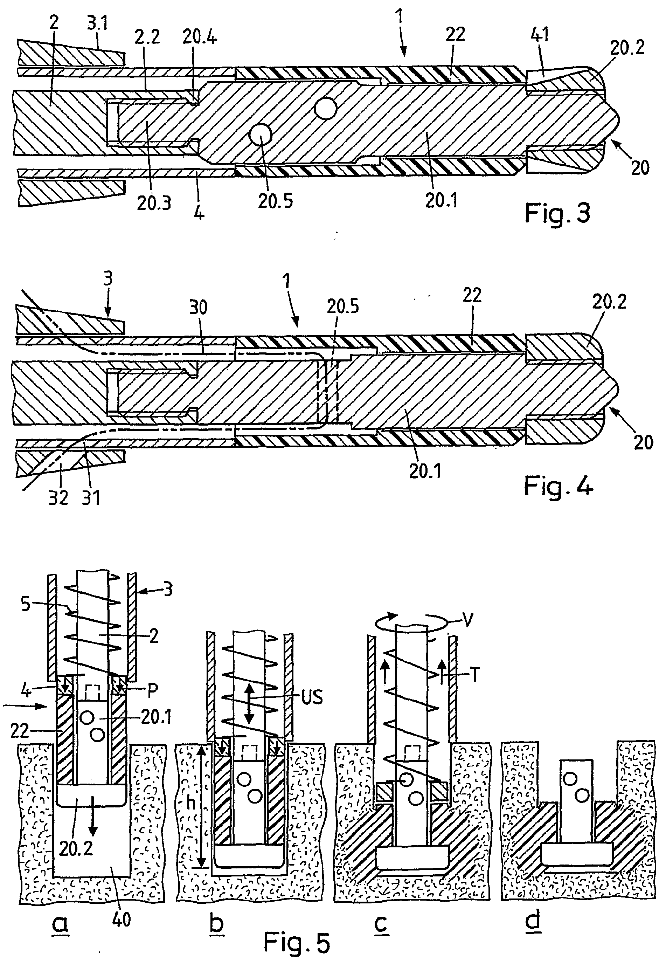

Figuren 3 und 4 zeigen den Anker 1 und seinen Anschluss an die Sonotrode 2 und die Stosshülse 4 in grösserem Massstab, wobei wiederum rechts das distale Ende der Vorrichtung dargestellt ist. Der Anker 1 weist einen Ankerfuss 20 und eine Verankerungshülse 22 auf. Der Ankerfuss 20 besteht beispielsweise aus einem Schaftteil 20.1 und einem auf dem Schaftteil aufgeschraubten Fussteil 20.2. Dabei hat der Fussteil 20.2 einen Durchmesser, der dem Aussendurchmesser der Stosshülse 4 im wesentlichen entspricht. Der Schaftteil 20.1 hat einen kleineren Durchmesser, derart, dass die proximale Stirnfläche des Fussteils 20.2 in seiner aufgeschraubten Position eine um den Schaftteil verlaufende Schulter bildet.

Auf der genannten Schulter steht die Verankerungshülse 22 mit ihrer distalen Stirnseite auf. Die Verankerungshülse 22 weist ein Lumen auf, das auf den Schaftteil 20.1 des Ankerfusses 20 abgestimmt ist, derart, dass die Hülse 22 lose auf dem Schaftteil sitzt. Das proximale Ende des Schaftteils 20.1 ragt vorteilhafterweise etwas aus der Verankerungshülse 22 hinaus und trägt einen Gewindezapfen 20.3 (oder anderes, geeignetes Verbindungsmittel), der in eine entsprechende Vertiefung mit Innengewinde am distalen Sonotrodenende 2.2 eingeschraubt ist, wodurch bezüglich der akustischen Funktion der Ankerfuss 20 Teil der Sonotrode 2 wird. An der Basis des Gewindezapfens 20.3 ist eine Sollbruchstelle 20.4 vorgesehen, die bei der Abtrennung der Sonotrode 2 vom verankerten Anker 1 nach dem Verankerungsprozess gebrochen wird, so dass der Gewindezapfen 20.3 zusammen mit der Sonotrode 2 vom verankerten Rest des Ankers getrennt werden kann.

Zwischenhülse 6, Stossfeder 5, Stosshülse 4 und Verankerungshülse 22, sind derart aufeinander und auf die Führungshülse 3 abgestimmt, dass bei distaler Position des Entspannungshebels 11 die Spannfeder 7 über die Zwischenhülse 6 die Stossfeder 5 derart spannt, dass diese die Stosshülse 4 und damit die Verankerungshülse 22 gegen den Ankerfuss 20 zu spannen vermag, auch dann noch, wenn die Verankerungshülse 22 sich durch die Verflüssigung des Hülsenmaterials verkürzt hat.

Der Faden (oder die Fäden) 30, der nur in der Figur 4 als strichpunktierte Linie angedeutet ist, liegt in einem mittleren Bereich in einer der Ösen 20.5, die im Schaftteil 20.1 des Ankerfusses 20 angeordnet sind. Die beiden Fadenteile, die beidseitig aus der Öse 20.5 ragen, verlaufen zwischen dem Schaftteil 20.1 des Ankerfusses 20 und der Verankerungshülse 22 in proximaler Richtung durch eine Fadenöffnung 31 in der Stosshülse 4 und eine weitere Fadenöffnung 32 in der Führungshülse 3. Von da verlaufen die beiden Teile des Fadens 30 in auf der Aussenseite der Führungshülse in axialer Richtung verlaufenden Fadennuten 33 (Fig. 1) und die Fadenenden sind in einer um den proximalen Teil 3.2 der Führungshülse verlaufenden Wickelnut 34 aufgewickelt oder in einem anderen, geeigneten Fadenmagazin, das Teil der Vorrichtung ist, untergebracht. Durch den Faden 30 wird die Sonotrode 2 und die Stosseinrichtung in der Führungshülse 3 gehalten, wobei der Faden eine beschränkte axiale Bewegung der Sonotrode relativ zur Führungshülse erlaubt. Zur Begrenzung einer Sonotrodenbewegung relativ zur Führungshülse 3 in distaler Richtung kann ein proximaler Bereich der Sonotrode 2 auch einen gegenüber dem distalen Bereich grösseren Querschnitt aufweisen (wie in Fig. 2 dargestellt), derart, dass dieser proximale Sonotrodenbereich nicht in das Lumen der Zwischenhülse 6 geschoben werden kann.

Wie aus den Figuren 3 und 4 ersichtlich ist, weist der Schaftteil 20.1 des Ankerfusses 20 im Bereich der Ösen 20.5 einen abgeflachten Querschnitt auf, so dass der Zwischenraum zwischen dem Schaftteil 20.1 und der Verankerungshülse 22 auf der Seite der Ösenmündungen breiter ist als auf den Seiten, die keine Ösenmündungen aufweisen. Durch diesen breiteren Zwischenraum läuft der Faden 30 (Figur 4), und durch die erhöhte Breite dieses Zwischenraumes wird verhindert, dass während des Verankerungsprozesses verflüssigtes Material der Verankerungshülse 22 mit dem Faden 30 in Kontakt kommt und dass dadurch die freie Verschiebbarkeit des Fadens durch die Öse 20.5 nach der Verankerung beeinträchtigt ist.

Die Sonotrode 2 besteht vorteilhafterweise aus Metall, beispielsweise aus Titan. Die Führungshülse 3 und die Zwischenhülse 6 bestehen beispielsweise aus Plexiglas, während für die Stosshülse 4 ein Material zu wählen ist, das bei der Verankerung möglichst wenig mit der Verankerungshülse 22 verklebt oder verschweisst wird, beispielsweise ebenfalls Titan oder, zur Erhöhung der Biegesteifigkeit des Hülsensystems, Chromstahl.

Die Sonotrode 2 ist akustisch derart ausgelegt, dass der Fussteil 20.2 des auf die Sonotrode aufgeschraubten Ankerfusses 20 mit maximaler, longitudinaler Amplitude schwingt. Dies wird erreicht, wenn die Sonotrode 2 mit Anker 1 eine Länge aufweist, die in etwa einer halben oder einer ganzen Wellenlänge der von der Schwingungsquelle gelieferten Schwingungen entspricht und die Schwingungsquelle derart eingerichtet ist, dass auch die Kopplungsstelle ein Ort maximaler longitudinaler Amplitude ist. Für die oben beschriebene Vorrichtung ergibt sich dabei beispielsweise für eine Frequenz von 30 kHz eine Sonotrodenlänge von etwa 180 mm (eine ganze Wellenlänge), was für die Verwendung der Vorrichtung zusammen mit einer für arthroskopische Eingriffe üblichen Kanüle sehr geeignet ist.

Die im Zusammenhang mit den Figuren 1 bis 4 beschriebene, erste Ausführungsform der erfindungsgemässen Vorrichtung stellt einen geschlossenen Lastrahmen dar, in dem die Stosseinrichtung (Elemente 4, 5, 6 und 7) und die Verankerungshülse 22 eingespannt sind zwischen der Führungshülse 3 und dem Ankerfuss 20, wobei die Führungshülse 3 während des Verankerungsprozesses auf dem Gewebe und am Gehäuse der Schwingungsquelle abgestützt ist und der Ankerfuss 20 an der Sonotrode befestigt ist, die ihrerseits im Gehäuse der Schwingungsquelle befestigt ist. Die bei der Verankerung auftretende Verkürzung der Verankerungshülse 22 wird durch eine entsprechende Verlängerung der Stossfeder 5 kompensiert.

Die erste Ausführungsform der erfndungsgemässen Vorrichtung hat die folgenden vier Konfigurationen:
- Lagerkonfiguration: die Sonotrode 2 ist gegenüber der Führungshülse 3 verdrehgesichert, (proximales Sonotrodenende in Stopfenelement 10), der Enspannungshebel 11 ist vorzugsweise in seiner proximalen Position (Spannfeder 7 komprimiert, Stossfeder 5 entspannt, nur geringe Kraft auf dem Anker 1). Der Faden ist zwischen Anker 1 und Wickelnut 34 derart gestreckt, dass er in der Fadennut 33 liegt. In dieser Konfiguration wird die Vorrichtung geliefert, gelagert und für den Verankerungsprozess an die Schwingungsquelle gekoppelt, wobei die Kraft für das Anschrauben an der Führungshülse 3 angreift.
- Kontrollkonfiguration: Das Distanzstück 34 (Fig. 2) wird zwischen proximalem Ende der Führungshülse 3 und dem Gehäuse der Schwingungsquelle eingebracht, beispielsweise aus dem Gehäuse ausgeschraubt, und damit die Sonotrode aus der Verdrehsicherung gezogen. Der Entspannunghebel 11 ist weiterhin in seiner proximalen Stellung. In dieser Konfiguration wird die Vorrichtung und deren Ankopplung an die Schwingungsquelle durch kurzes Einschalten der Schwingungsquelle geprüft und eingestimmt. Wenn die von der Schwingungsquelle aufgenommene Leistung in einem vorgegebenen Bereich liegt, arbeitet die Vorrichtung korrekt. Da auf die Verankerungshülse 22 keine wesentliche Kraft wirkt, erfolgt in dieser Konfiguration keine Verflüssigung des Hülsenmaterials.
- Verankerungskonfiguration: der Enspannungshebel 11 wird aus seiner proximalen Position gelöst und durch die Spannfeder 7 in seine distale Position gebracht, wodurch die Stossfeder 5 gespannt wird. In dieser Konfiguration wird das distale Ende der Vorrichtung in einer im Gewebe vorbereiteten Öffnung positioniert, derart, dass die distale Stirnseite der Führungshülse 3 auf der Gewebeoberfläche abgestützt ist und am Distanzstück 34 anschlägt. Wenn notwendig, wird durch Verstellen des Distanzelementes oder gegebenenfalls durch Ein- oder Ausschrauben des distalen Führungshülsenteils 3.1 in den oder aus dem proximalen Führungshülsenteil 3.2 die Verankerungstiefe (Distanz zwischen distalem Ende des Ankers 1 und distaler Stirnseite der Führungshülse 3 bzw. Gewebeoberfläche) angepasst. Wenn notwendig, wird der Faden nachgespannt. Durch Einschalten der Schwingungsquelle wird die Verankerung gestartet.
- Trennkonfiguration: Der Faden wird von der Führungshülse 3 gelöst. In dieser Konfiguration wird nach Beendigung der Verankerung die Sonotrode 2 durch eine leichte Drehung vom verankerten Anker 1 gelöst, wobei die Sollbruchstelle am Schaftteil 20.1 des Ankerfusses 20 bricht, so dass die Sonotrode 2 mit der Führungshülse 3 und der Stosseinrichtung vom verankerten Anker entfernt werden kann.

**Figur 5** zeigt in einer mehr schematischen Art vier aufeinander folgende Phasen (a bis d) eines Verankerungsprozesses mit Hilfe der ersten Ausführungsform der erfindungsgemässen Vorrichtung. Dabei sind nur der Anker 1 und die distalen Bereiche von Sonotrode 2, Führungshülse 3 und Stosseinrichtung (Stosshülse 4 und Stossfeder 5) dargestellt.

In Phase a wird der das distale Ende der erfindungsgemässen Vorrichtung bildende Anker 1 in einer entsprechend vorbereiteten Gewebeöffnung 40 positioniert, wobei die Tiefe der Gewebeöffnung 40 und die Verankenungstiefe h derart aneinander angepasst sind, dass der Fussteil des Ankerfusses nicht auf dem Öffnungsgrund aufsteht. Die Vorrichtung ist in der Verankerungs-Konfiguration, das heisst die Stosshülse 4 wird durch die gespannte Stossfeder 5 gegen die Verankerungshülse 22 und den Fussteil 20.2 des Ankerfusses gepresst (Pfeile P).

In Phase b ist der Anker 1 positioniert (distale Stirnseite der Führungshülse 3 steht auf der Gewebeoberfläche auf) und die Schwingungsquelle wird eingeschaltet (Doppelpfeil US). Dadurch wird in Phase c das Material der Verankerungshülse 22 verflüssigt und interpenetriert das benachbarte Gewebe. Wenn eine vorgegebene Verankerungszeit abgelaufen oder eine vorgegebene Verkürzung der Verankerungshülse 22 erreicht ist, wird die Schwingungsquelle abgeschaltet. Es ist aber auch möglich, dass der Chirurg ad hoc die Verankerungszeit oder, anhand entsprechender Markierungen an Sonotrode und (transparenter) Führungshülse, den Verankerungshub und damit die Menge des verflüssigten Material frei wählt.

In Phase c wird die Trennkonfiguration erstellt, das heisst der Faden wird von der Führungshülse getrennt und die Sonotrode 2 bzw. die Schwingungsquelle wird für die Abtrennung des Gewindebolzens gedreht (Pfeil V) und die Schwingungsquelle wird mit Sonotrode 2, Führungshülse 3 und Stosseinrichtung entfernt (Pfeile T). In Phase d ist der Anker fertig verankert und vom Rest der Vorrichtung getrennt.

Aus Fig. 5 ist ersichtlich, dass die Tiefe des Ankers im Gewebe bei der Ankerpositionierung festgelegt und während der Verankerung nicht mehr verändert wird. Dies stellt einen Vorteil gegenüber herkömmlichen, eingeschraubten und eingeschlagenen Ankern dar, deren Tiefe und Verankerung im Gewebe untrennbar miteinander verkoppelt sind.

Wie in der Figur 5 dargestellt ist, wird das Material der Verankerungshülse 3 durch die von der Sonotrode 2 auf den Ankerfuss 20 übertragenen Vibrationen vor allem an der Kontaktfläche zwischen Ankerfuss 20 und Verankerungshülse 22 verflüssigt. Je nach Materialpaarung und Ausgestaltung der Kontaktflächen kann auch eine zusätzliche oder exklusive Verflüssigung an Kontaktflächen zwischen Verankerungshülse 22 und Stosshülse 4 bewirkt werden. Generell gilt, dass von der Vibration des Ankerfusses 20 nur ein kleiner Teil in die Verankerungshülse 22 übertragen wird und dass aus diesem Grunde bei gleichen Materialien und gleichen Ausgestaltungen der Kontaktflächen die Verflüssigung hauptsächlich in Kontakt mit dem Ankerfuss 20 stattfmdet. Für eine zusätzliche Verflüssigung im Kontakt mit der Stosshülse 4 ist dort ein verflüssigbares Material mit einer relevant niedrigeren Glasumwandlungstemperatur vorzusehen und/oder die Kontaktfläche zwischen Verankerungshülse 22 und Stosshülse 4 ist relevant zu verkleinern, beispielsweise durch eine Zuspitzung der Verankerungshülse 22 oder durch auf der Kontaktfläche angeordnete Energierichtungsgeber in der Form von vorstehenden Rippen oder Höckern. Es hat sich herausgestellt, dass sich insbesondere ein Muster von radial verlaufenden Rippen und Kämmen für die Funktion der Energierichtungsgeber eignet.

Es ist auch möglich, die Verankerungshülse 22 zweiteilig oder mehrteilig auszugestalten, wodurch bei entsprechender Ausgestaltung der Kontaktflächen innerhalb der Verankerungshülse 22 auch beispielsweise in einem mittleren Hülsenbereich eine Verflüssigung erreicht werden kann. Wie bereits weiter oben angetönt, kann die Verankerungshülse nicht runde innere und/oder äussere Querschnitte aufweisen. Es ist auch keine Bedingung, dass alle Querschnitte dieser Verankerungshülse einen geschlossenen Ring darstellen. Die Hülse kann ohne weiteres ganz oder teilweise geschlitzt sein.

Während eine Verschweissung zwischen Ankerfuss und Verankerungshülse gegebenenfalls erwünscht ist, soll eine derartige Verschweissung zwischen Verankerungshülse und Stosshülse möglichst verhindert werden. Eine Verschweissung wird üblicherweise erreicht, wenn beide Materialien Polymere sind und ihre Glasumwandlungstemperaturen nicht mehr als ca. 50°C auseinander liegen. Eine Verschweissung wird verhindert, wenn das Material von Ankerfuss 20 und/oder Stosshülse 4 unter den Bedingungen der Verankerung nicht verflüssigbar ist (Metall oder Keramik) und vom verflüssigten Material der Verankerungshülse 22 nicht benetzbar ist.

Wenn der Ankerfuss 20 bei der Verankerung mit der Verankerungshülse 22 nicht verschweisst wird, ist es vorteilhaft, in der Schulter des Ankerfusses 20 bzw. des Fussteils 20.2 Ausnehmungen vorzusehen, in die das verflüssigte Material fliesst, so dass nach dem Wiedererstarren des verflüssigten Materials der Ankerfuss 20 und damit auch die Fadenösen 20.5 gegen Verdrehung in der Verankerungshülse 22 gesichert werden. Solche Ausnehmungen sind in der Figur 3 dargestellt und mit 41 bezeichnet.

Die erste Ausführungsform der erfindungsgemässen Vorrichtung kann beispielsweise dadurch variiert werden, dass das Stopfenelement 10 einen runden Sicherungsteil enthält, der die Austrittsöffnung für die Sonotrode aufweist und über eine Sollbruchstelle im Stopfenelement befestigt ist. Dabei ist die Sollbruchstelle derart ausgelegt, dass sie beim Anschrauben der Sonotrode an die Schwingungsquelle bricht, sobald die Verschraubung fest ist.

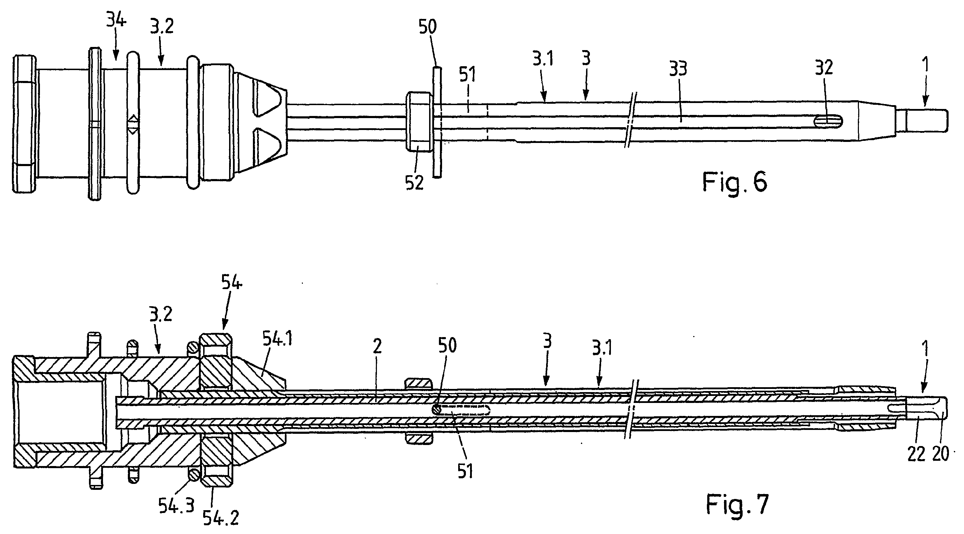

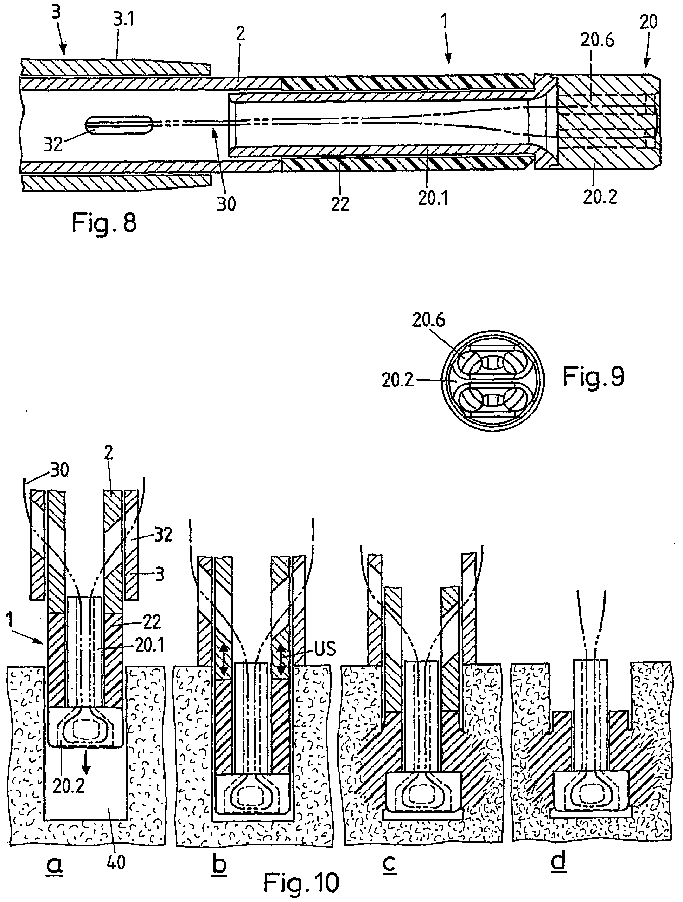

**Figuren 6 bis 9** illustrieren eine zweite, beispielhafte Ausführungsform der erfindungsgemässen Vorrichtung, wobei Fig. 6 eine Ansicht der ganzen Vorrichtung und Fig. 7 einen axialen Schnitt durch die ganze Vorrichtung zeigen und wobei Figuren 8 und 9 in einem grösseren Massstab einen axialen Schnitt durch den Anker und eine Draufsicht auf das distale Ende des Ankers zeigen.

Die Vorrichtung weist wiederum einen Anker 1 am distalen Vorrichtungsende, eine Sonotrode 2, eine Führungshülse 3 und einen Faden 30 (strichpunktierte Linie in Fig. 8) auf, wobei die Sonotrode 2 sich vom Anker 1 durch die Führungshülse 3 bis in deren proximalen Teil erstreckt. Die Führungshülse 3 hat einen distalen Teil 3.1 mit einem kleineren Querschnitt und einen proximalen Teil 3.2 mit einem grösseren Querschnitt, wobei die beiden Hülsenteile fest miteinander verbunden, ineinander geschraubt oder als ein Stück gefertigt sind. Der Anker 1 hat wiederum einen Ankerfuss 20 und eine Verankerungshülse 22, wobei die Verankerungshülse 22 das verflüssigbare Material aufweist oder aus diesem besteht und während des Verankerungsprozesses verkürzt wird. Im Gegensatz zur ersten Ausführungsform weist die zweite Ausführungsform der erfindungsgemässen Vorrichtung keine Stosseinrichtung auf, so dass es möglich ist, den Querschnitt des distalen Vorrichtungsbereichs weiter zu verkleinern. Weitere Unterschiede bestehen darin, dass die Vorrichtung keinen geschlossenen Lastrahmen darstellt, dass für die Verkürzung der Verankerungshülse ein Hub der Schwingungsquelle notwendig ist und dass der Faden bei der Verankerung eine mehr aktive Funktion übernimmt.

Die Sonotrode 2 ist mindestens in ihrem distalen Bereich rohrförmig mit auf die Verankerungshülse 22 abgestimmten Durchmessern und in ihrem proximalen Bereich ist sie für eine Verbindung mit der Schwingungsquelle (nicht dargestellt) beispielsweise mit einem Innengewinde versehen, wobei die Anschlussstelle des Schwingungsquelle derart ausgestaltet ist, dass sie eine beschränkte axiale Bewegung (Verankerungshub) der Sonotrode 2 relativ zur Führungshülse 3 erlaubt, ohne dass die Führungshülse am Gehäuse der Schwingungsquelle anstösst.

Die Sonotrode 2 und die Führungshülse 3 sind wirkverbunden durch einen Verbindungsbolzen 50, der in einer radialen Bohrung in der Sonotrode steckt und für den in der Führungshülse 3 einander gegenüberliegende, axial verlaufende, schlitzförmige Bolzenöffnungen 51 vorgesehen sind, deren axiale Ausdehnung mindestens im proximalen Bereich durch einen auf dem distalen Führungshülsenbereich 3.1 aufgeschraubten Einstellring 52 einstellbar sein kann. Durch die Schraubposition des Einstellrings 52 wird die Verankerungstiefe (Distanz zwischen Gewebeoberfläche und distalem Ende des Ankers 1) definiert.

Am distalen Ende des proximalen Führungshülsenteils 3.2 ist eine Fadenspannvorrichtung 54 angeordnet, die beispielsweise einen Fadenspannring 54.1, einen Übergangsring 54.2 und einen Klemmring 54.3 umfasst, wobei der Fadenspannring 54.1 auf dem distalen Führungshülsenteil 3.1 aufgeschraubt und dadurch axial verstellbar ist und wobei der Klemmring, wenn er gegen den Übergangsring gezogen wird, den ihn umschlingenden, gespannten Faden selbsthemmend zu halten vermag.

Aus den Figuren 8 und 9 ist ersichtlich, dass der Fussteil 20.2 des Ankerfusses 20 pro Faden zwei axial verlaufende Fadenbohrungen 20.6 (dargestellt sind vier Bohrungen für zwei Fäden) aufweist und der Schaftteil 20.1 des Ankerfusses 20 rohrförmig ausgebildet ist und einen verbreiterten distalen Bereich aufweist. Dieser distale Bereich des Schaftteils 20.1 bildet die Schulter, auf der die Verankerungshülse 22 aufsteht. Ferner ist dieser distale Bereich derart an den proximalen Bereich des Fussteils 20.2 angepasst, dass die beiden Teile durch Ineinanderschnappen aneinander befestigt werden können.

Ein mittlerer Bereich des Fadens 30, der nur in Figur 8 als strichpunktierte Linie teilweise dargestellt ist, verläuft durch zwei der Fadenbohrungen 20.6 im Fussteil 20.2 des Ankerfusses 20 und wird dadurch im Anker gehalten. Die beiden proximal aus den Fadenbohrungen 20.6 ragenden Fadenteile verlaufen durch den Schaftteil 20.1 des Ankerfusses und in den distalen Bereich der Sonotrode 2 von wo sie durch aufeinander ausgerichtete Fadenöffnungen 32 in der Sonnotrode 2 und der Führungshülse 3 an die Aussenseite der Führungshülse geführt sind. Von da verlaufen sie in Fadennuten 33 durch den Einstellring 52 hindurch in die Fadenspannvorrichtung 54, das heisst, über den Fadenspannring 54.1 durch entsprechende axial verlaufende Öffnungen im Übergangsring 54.2, um den Klemmring 54.3 herum und zurück durch den Übergangsring 54.2. Durch Zug an den Fadenenden, die aus dem Übergangsring 54.2 ragen, wird der Faden 30 gespannt und der Klemmring 54.3 gegen den Übergangsring 54.2 gezogen, wodurch eine selbsthemmende Fadenhalterung erstellt wird. Für ein Nachspannen kann der Fadenspannring in eine weiter distal liegende Position geschraubt werden. Die Fadenenden sind in der Wickelnut 34 (Fadenmagazin) aufgewickelt.

Die Spannung des Fadens 30, die in der zweiten Ausführungsform der erfindungsgemässen Vorrichtung ein relevanter Parameter ist, ist derart, dass der Faden ohne wesentliche elastische Verlängerung die durch die Sonotrode auf die Verankerungshülse 22 auszuübende Presskraft aufnehmen kann, so dass eine Verflüssigung des Materials der Verankerungshülse 22 und eine Verkürzung dieser Hülse möglich werden.

Die Materialien für die einzelnen Elemente der Vorrichtung gemäss Figuren 6 bis 9 entsprechen den für die erste Ausführungsform weiter oben beschriebenen Materialien.

Die Sonotrode 2 ist akustisch derart ausgelegt, dass ihr distales Ende mit einer maximalen longitudinalen Amplitude schwingt. Wenn die Kopplungsstelle der Schwingungsquelle ebenfalls mit einer maximalen longitudinalen Amplitude schwingt, ist die Sonotrodenlänge beispielsweise so gross wie eine halbe oder eine ganze Wellenlänge der durch die Schwingungsquelle erzeugten Schwingung im Sonotrodenmaterial. Der Verbindungsbolzen 50 ist in einem Knotenpunkt anzuordnen, also, bei einer Sonotrode 2 mit der Länge einer ganzen Wellenlänge um ¾ der Wellenlänge vom distalen Sonotrodenende entfernt.

Die zweite Ausführungsform der erfindungsgemässen Vorrichtung hat drei Konfigurationen:
- Lager- und Kontrollkonfiguration: der Faden ist nur derart gespannt, dass der Anker ausreichend auf der Sonotrode hält und der Verbindungsbolzen 50 in seiner proximalsten Stellung ist. In dieser Konfiguration wird die Vorrichtung geliefert, gelagert und an die Spannungsquelle gekoppelt, wobei die für ein Anschrauben notwendige Kraft beispielsweise am Verbindungsbolzen 50 angreift. In dieser Konfiguration wird für die Betriebskontrolle und Einstimmung die Schwingungsquelle kurz eingeschaltet, wobei die Sonotrode 2 nur durch die Fadenspannung gegen die Verankerungshülse gepresst wird, was für eine Verflüssigung des Verankerungshülsenmaterials nicht reicht. Wenn die von der Schwingungsquelle aufgenommene Leistung in einem vorgegebenen Bereich liegt, ist die Vorrichtung betriebsbereit.
- Verankerungskonfiguration: Vor oder nach dem Einbringen des distalen Endes der Vorrichtung in die Gewebeöffnung (Führungshülse 3 auf Gewebeoberfläche abgestützt) kann die Verankerungstiefe durch Verschiebung des Einstellrings 52 angepasst und gegebenenfalls der Faden (zur Erhöhung der Stabilität des Ankers auf der Sonotrode) durch Verdrehung das Fadenspannrings 54.1 nachgespannt werden. In dieser Konfiguration wird die Sonotrode gegen den Anker 1 gepresst, die Schwingungsquelle eingeschaltet und dadurch der Anker 1 verankert.
- Trennkonfiguration: die Fadenspannvorrichtung wird gelöst durch eine Verschiebung des Klemmrings 54.3 gegen das proximale Ende der Vorrichtung. In dieser Konfiguration wird der verankerte Anker von den anderen Bestandteilen der Vorrichtung getrennt, wozu diese lediglich wegbewegt werden müssen.

**Figur 10** zeigt in der gleichen Weise wie Figur 5 vier aufeinanderfolgende Phasen a bis d des Verankerungsprozesses mit Hilfe der zweiten Ausführungsform der erfindungsgemässen Vorrichtung.

In Phase a wird das distale Ende der Vorrichtung bzw. der Anker 1 in der Gewebeöffnung positioniert. Die definitive Position des Ankers 1 im Gewebe ist erreicht, wenn die distale Stirnseite der Führungshülse 3 auf der Gewebeoberfläche aufsteht, was in Phase b der Fall ist. Jetzt wird die Schwingungsquelle eingeschaltet (Doppelpfeile US) und die Sonotrode 2 gegen die Verankerungshülse 22 gedrückt, wodurch das Material der Hülse verflüssigt wird und radial ausfliesst und wodurch die Verankerungshülse 22 kürzer wird. Die weiter pressende Sonotrode 2 wird dadurch gegenüber der auf der Gewebeoberfläche aufstehenden Führungshülse 3 verschoben, wie dies in Phase c dargestellt ist. Der Anker 1 bleibt durch den Faden 30 gehalten stationär. Der Hub der Sonotrode 2 kann begrenzt sein durch ein Anschlagen des proximalen Endes der Führungshülse 3 am Gehäuse der Schwingungsquelle oder durch das distale Ende der Bolzenöffnungen 51 gegen die der Verbindungsbolzen 50 sich während des Verankerungsprozesses bewegt. Für eine Einstellung des Verankerungshubes kann im Bereich dieses distalen Endes auch ein weiterer Einstellring (nicht dargestellt) vorgesehen werden, der je nach durchzuführender Verankerung die Bolzenöffnungen 51 mehr oder weniger weit distal abschliesst. Phase 4 zeigt den fertig verankerten Anker 1, von dem nach dem Lösen des Fadens 30 aus der Spannvorrichtung 54 die restlichen Teile der Vorrichtung getrennt worden sind.

Wie bereits im Zusammenhang mit der ersten Ausführungsform der erfindungsgemässen Vorrichtung weiter oben beschrieben, wird bei gleichem Material und gleichen Kontaktflächen zwischen Verankerungshülse 22 und Sonotrode 2 und zwischen Verankerungshülse 22 und Ankerfuss 20, das Hülsenmaterial vornehmlich an der Kontaktfläche mit der Sonotrode 2 verflüssigt. Eine vornehmliche Verflüssigung an der Kontaktfläche zum Ankerfuss 20, wie in der Figur 10 dargestellt, wird beispielsweise erreicht, indem die Verankerungshülse 22 auf dieser Seite eine spitz zulaufende Stirnseite aufweist, wie dies in der Figur 8 dargestellt ist. Dasselbe könnte erreicht werden, indem die Verankerungshülse 22 an ihrem distalen Ende ein Material aufweist mit einer relativ tiefen Glasumwandlungstemperatur, während am proximalen Ende ein nicht verflüssigbares Material angeordnet ist oder ein Thermoplast mit einer höheren Glasumwandlungstemperatur.

Die zweite Ausführungsform der erfindungsgemässen Vorrichtung kann beispielsweise dadurch variiert werden, dass der Druck der Sonotrode 2 auf die Verankerungshülse 22 und der Verankerungshub nicht durch eine Bedienungsperson erzeugt wird, sondern dass die Vorrichtung einen geschlossenen Lastrahmen bildet, in dem die Sonotrode 2 derart gegen die Verankerungshülse 22 gespannt ist, dass der notwendige Druck für die Verankerung über den ganzen notwendigen Hub aufrechterhalten wird. Wie für die erste Ausführungsform kann dies auch für die zweite Ausfuhrungsform realisiert werden durch eine Stossfeder, die beispielsweise in vorgespanntem Zustand zwischen dem proximalen Führungshülsenteil 3.2 und dem Verbindungsbolzen 50 eingespannt ist und die den Verbindungsbolzen 50 gegen das distale Ende der Bolzenöffnungen 51 drückt. Dabei müsste der Faden stark genug sein, um die Federkraft aufzunehmen und für die Kontrollkonfiguration müsste die Feder in einer geeigneten Weise inaktiv geschaltet werden können.

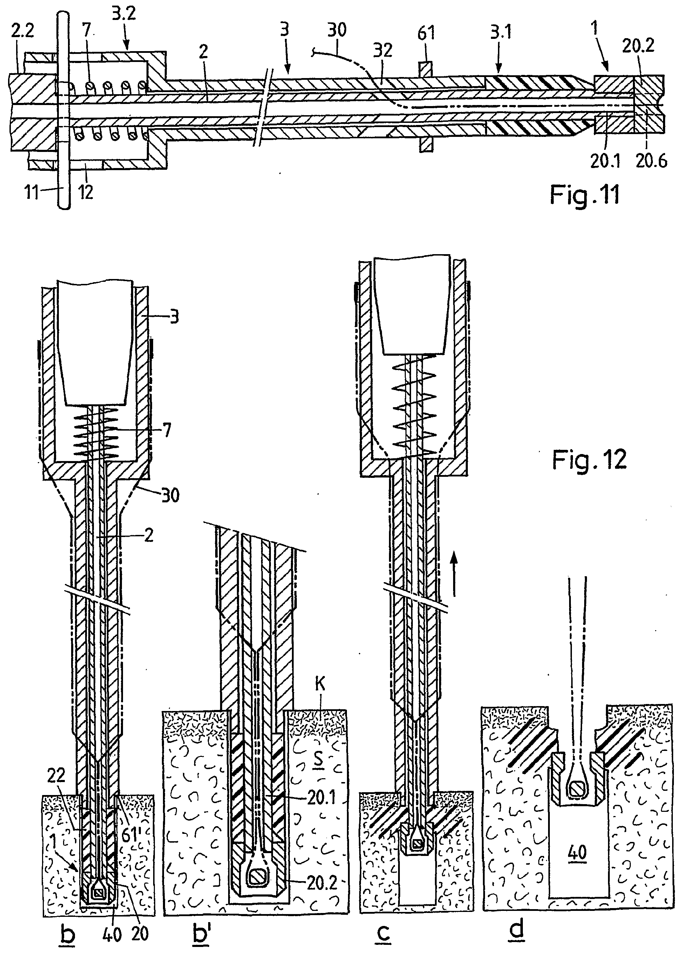

Der Verankerungsprozess unter Verwendung der zweiten Ausführungsform der erfindungsgemässen Vorrichtung kann auch folgendermassen ablaufen: Die Führungshülse ist während des Prozesses an der Schwingungsquelle abgestützt, so dass die Schwingungsquelle und damit die Sonotrode nicht gegen das Gewebe verschoben werden können. Durch entsprechenden Zug am Faden wird die für die Verflüssigung des Verankerungshülsenmaterials und den Verankerungshub notwendige Kraft erzeugt und der Verankerungshub wird vom Ankerfuss ausgeführt, wobei sich der Ankerfuss gegen die Gewebeoberfläche verschiebt. Eine solches Verfahren eignet sich speziell für eine Verankerung an der inneren Seite der Kortikalis, also für eine Verankerung in einem Knochen, in dem die Spongiosa fehlt oder mechanische Eigenschaften aufweist, die für eine Verankerung nicht ausreichen. Der Abstand zwischen den distalen Enden der Führungshülse 3 und der Sonotrode 2 wird in diesem Falle auf die Dicke der Kortikalis abgestimmt. Eine gleiche Verankerung, die aber mit Hilfe einer anderen Vorrichtung erstellt wird, ist in der Figur 12 dargestellt und wird weiter unten im Zusammenhang mit dieser Figur im Detail beschrieben.

**Figur 11** zeigt eine dritte beispielhafte Ausführungsform der erfindungsgemässen Vorrichtung in einem axialen Schnitt. Diese Ausführungsform verbindet Merkmale der ersten und der zweiten Ausführungsform, insbesondere der Variante der zweiten Ausführungsform mit geschlossenem Lastrahmen, welche Variante weiter oben zusätzlich zu den Figuren beschrieben ist und in welcher Variante eine Feder die Funktion der Spannung und des Hubes übernimmt. Im Unterschied zur genannten Variante der zweiten Ausführungsform führt aber in der dritten Ausführungsform die Sonotrode während des Verankerungsprozesses einen Hub vom Gewebe weg aus und der Ankerfuss ist fest mit dem distalen Sonotrodenende verbunden, wie dies für die erste Ausführungsform der Fall ist.

Die dritte Ausführungsform der erfindungsgemässen Vorrichtung weist wiederum einen Anker 1, eine Sonotrode 2, eine Führungshülse 3 und einen Faden 30 auf, wobei die Führungshülse neben der Führungshülsenfunktion der ersten und zweiten Ausführungsform auch die Funktion einer stationären Stosseinrichtung übernimmt. Die Führungshülse 3 weist wiederum einen distalen Teil 3.1 mit kleinerem Querschnitt und einen proximalen Teil 3.2 mit grösserem Querschnitt auf. Am distalen Teil 3.1 trägt die Führungshülse einen beispielsweise mit Hilfe eines Gewindepaars (nicht dargestellt) auf der Führungshülse 3 axial verschiebbaren Stellring 61 oder einen entsprechenden, festen Flansch (oder eine stufenartige Querschnittsverkleinerung) auf, wobei die axiale Position des Stellrings 61, oder des Flansches die Verankerungstiefe (Distanz zwischen proximalem Ende des Ankers und Gewebeoberfläche) bestimmt.

Das proximale Ende der Sonotrode 2 ist wiederum ausgerüstet für eine Kopplung an die Schwingungsquelle und weist einen Kopf 2.2 mit einem gegenüber dem Rest der Sonotrode 2 vergrösserten Querschnitt auf. Die Sonotrode 2 ist mindestens in einem distalen Bereich rohrförmig und für eine Befestigung des Ankerfusses 20 an der Sonotrode 2 ausgerüstet, derart, dass der Ankerfuss 20 einen Teil des akustischen Systems bildet. Diese Verbindung muss für eine Trennung der Sonotrode 2 vom Ankerfuss 20 nach der Verankerung lösbar sein und ist beispielsweise realisiert als Aussengewinde aus Metall auf der Sonotrodenseite und Innengewinde aus einem Thermoplasten auf der Seite des Ankerfusses 20. Dabei ist der Ankerfuss 20 derart ausgelegt, dass er sich mindestens im Bereich des Innengewindes während des Verankerungsprozesses erwärmt, so dass die Sonotrode 2 am Ende des Verankerungsprozesses durch einen kleinen Ruck unter Zerstörung des Innengewindes vom Ankerfuss 20 getrennt werden kann. Es kann für die Trennung auch eine geeignete Sollbruchstelle auf der Seite des Ankerfusses 20 vorgesehen werden, wie dies für die erste Ausführungsform der erfindungsgemässen Vorrichtung weiter oben beschrieben ist.

Die Sonotrode 2 ist gegen die Führungshülse 3 gespannt durch eine zwischen dem Sonotrodenkopf 2.2 und einer inneren Schulter des proximalen Führungshülsenteils 3.2 vorgespannten Spannfeder 7, die den Sontotrodenkopf 2.2 in proximaler Richtung aus der Führungshülse drückt. Zur Entlastung der Sonotrode 2 von der Federkraft (Kontrollkonfiguration) ist analog zur ersten Ausführungsform ein mit der Spannfeder 7 wirkverbundener Entspannungshebel 11 vorgesehen, dessen mittlerer Teil von der Sonotrode 2 durchdrungen wird und deshalb unabhängig von dieser bewegbar ist. Die Bewegung des Entspannungshebels ist geführt in sich axial erstrekkenden Öffnungen 12 in der Führungshülse 3, welche Öffnungen an ihrem distalen Ende eine senkrecht zur Vorrichtungsachse ausgerichtete Verlängerung (nicht dargestellt) aufweisen, in die der Entspannungshebel 11 zur Entlastung der Sonotrode eingerastet werden kann. Wenn der Entspannungshebel 11 nicht eingerastet ist, wird er durch die Spannfeder 7 gegen den Sonotrodenkopf 2.2 gedrückt.

Der Anker 1 weist wiederum einen Ankerfuss 20 auf, der für diese Ausführungsform einteilig oder wiederum zweiteilig (mit Schaftteil 20.1 und Fussteil 20.2) sein kann, sowie eine Verankerungshülse 22 aus dem verflüssigbaren Material. Der Faden 30, dessen mittlerer Bereich beispielsweise durch zwei axiale Bohrungen im Fussteil 20.2 des Ankerfusses 20 geführt ist, verläuft durch den Schaftteil 20.1 in die rohrförmige Sonotrode 2 und durch aufeinander abgestimmte Fadenöffnungen 32 in Sonotrode 2 und Führungshülse 3 auf die Aussenseite der Führungshülse 3, von wo die Fadenenden in proximaler Richtung zu einem in der Vorrichtung integrierten Fadenmagazin (nicht dargestellt) geführt sind. Da der Faden vom Ankerfuss 20 unmittelbar in die Sonotrode läuft, kann das distale Sonotrodenende die den Faden vor verflüssigtem Material schützende Funktion des Schaftteiles 20.1 des Ankerfusses übernehmen und der Schaftteil 20.1 des Ankerfusses kann fehlen.

Die Vorrichtung stellt einen geschlossenen Lastrahmen dar, in dem die Verankerungshülse 22 zwischen dem Ankerfuss 20 und der distalen Stirnseite der Führungshülse 3 verspannt ist, dadurch dass der Ankerfuss 20 am distalen Ende der Sonotrode 2 befestigt ist und die Sonotrode 2 durch die Spannfeder 7 gegen die Führungshülse verspannt ist, derart, dass der Druck auf die Verankerungshülse 22 ausreicht um unter dem Einfluss der Vibrationen der Schwingungsquelle das Material der Verankerungshülse 22 zu verflüssigen und die Verankerungshülse zu verkürzen. Diese Verkürzung wird durch eine Verlängerung der Spannfeder und einen entsprechenden Hub der Sonotrode kompensiert. Der Faden hat keine Funktion bei der Verankerung, das heisst, er kann, muss aber nicht, an der Führungshülse befestigt sein.

Die dritte Ausführungsform der erfindungsgemässen Vorrichtung kann beispielsweise variiert werden, indem die Spannfeder 7 weggelassen wird und die Spannkraft und der Verankerungshub durch Ziehen an der Schwingungsquelle oder am Faden durch eine Bedienungsperson erbracht werden, wie dies in analoger Weise auch für die zweite Ausführungsform weiter oben beschrieben ist. Ferner kann die dritte Ausführungsform der erfindungsgemässen Vorrichtung variiert werden, indem anstelle des Stellrings 61 oder festen Flansches eine weitere Hülse vorgesehen wird, die aussen an der Führungshülse angeordnet und in geeigneter Weise axial gegen diese verschiebbar ist.

**Figur 12** zeigt drei aufeinander folgende Phasen einer Verankerung, die in Anlehnung an die Figuren 5 und 10 mit b, c und d bezeichnet sind, wobei mit b' eine grössere Darstellung der Phase b bezeichnet ist. Die Verankerung wird mit Hilfe einer Variante der Vorrichtung nach Figur 11 ausgeführt. In Phase b ist das distale Vorrichtungsende bzw. der Anker 1 in einer Gewebeöffnung 40 positioniert, so dass eine im distalen Bereich der Führungshülse angeordnete Stufe 61' (Funktion wie Stellring 61 in Figur 11) auf der Gewebeoberfläche aufsteht. Die Schwingungsquelle wird eingeschaltet. Dadurch wird die Sonotrode 2, getrieben durch die Spannfeder 7 und ermöglicht durch die Verflüssigung des Materials der Verankerungshülse 22, in proximaler Richtung bewegt, während die Führungshülse 3 stationär bleibt. Das verflüssigte Material fliesst in das umgebende Gewebe. Zur Trennung des verankerten Ankers (Phase d) muss lediglich der Faden 30 von der Führungshülse 3 gelöst und die Sonotrode 2 vom Ankerfuss 22 getrennt und zusammen mit der Führungshülse 3 entfernt werden.

Wie bereits weiter oben erwähnt, eignet sich die in der Figur 12 dargestellte Ausführungsform insbesondere für eine Verankerung auf der inneren Seite der Kortikalis K (subkortikale Verankerung), was insbesondere dann vorteilhaft ist, wenn die Spongiosa S fehlt oder mechanische Eigenschaften aufweist, die für eine Verankerung völlig ungenügend sind. Wie in der Figur 12 dargestellt, entspricht für eine solche Anwendung der Abstand zwischen der Stufe 61' und dem distalen Ende der Führungshülse 3 der Dicke der Kortikalis K und die Verankerungshülse 22 ist relativ kurz. Während des Verankerungsvorgangs, in dem das distale Ende der Verankerungshülse 22 aufgeschmolzen wird, wird die Verankerungshülse verkürzt und nähert sich in einer letzten Phase der inneren Seite der Kortikalis, an der ein wesentlicher Teil der Verankerung entsteht. Der Ankerfuss 20 besteht beispielsweise aus PEEK oder aus PLDLA, während die Verankerungshülse 22 aus PLDLA besteht und während des Verankerungsvorgangs auch mit dem Ankerfuss verschweisst wird.

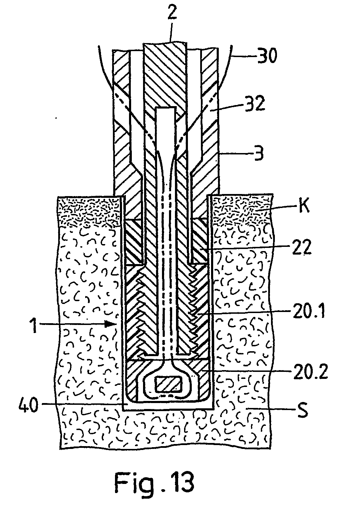

Figur 13 stellt eine weitere Variante eines Ankers 1 dar, der sich eignet für die Vorrichtung gemäss Figur 11 und das Verfahren gemäss Figur 12 und der das distale Ende einer erfindungsgemässen Vorrichtung bildend und in einer Gewebeöffnung 40 positioniert dargestellt ist. Elemente mit gleichen Funktionen sind mit gleichen Bezugsziffen bezeichnet wie in den vorhergehenden Figuren.

Der Anker 1 weist wiederum einen Ankerfuss 20 und eine Verankerungshülse 22 auf, wobei der Ankerfuss 20 einen Schaftteil 20.1 und einen Fussteil 20.2 aufweist. Der Schaftteil 20.1 weist im wesentlichen einen gleichen Querschnitt auf wie die Verankerungshülse und seine proximale Stirnseite ist die Schulter auf der die Verankerungshülse aufsteht. Der Schaftteil 20.1 ist beispielsweise wie dargestellt mittels Innengewinde am distalen Sonotrodenende befestigt, der Fussteil 20.2 wird beispielsweise durch den Faden 30 in seiner Position gehalten oder ist auf den Schaftteil aufgeschraubt. Der Fussteil 20.2 und der Schaftteil 20.1 bestehen beispielsweise aus PEEK, während die Verankerungshülse aus PLDLA besteht. Es ist aber auch möglich, alle drei Ankerteile aus PLDLA herzustellen. In jedem Falle ist die Befestigung des Sonotrodenendes im Schaftteil 20.1 derart auszugestalten, dass sie nach der Verankerung gelöst (gegebenenfalls unter Zerstörung) werden kann. Vorteilhafterweise sind die Materialien derart zu wählen, dass bei der Verankerung der Schaftteil 20.1 mit der Verankerungshülse und gegebenenfalls mit dem Fussteil 20.2 verschwqeisst wird, was bei einer Materialpaarung PEEK/PLDLA der Fall ist.

In den obigen Abschnitten sind drei Ausführungsformen der erfindungsgemässen Vorrichtung mit mehr oder weniger vielen Details beschrieben, sowie einige weitere Varianten davon angetönt. Es ist für den Fachmann ohne weiteres möglich, Merkmale einer Ausführungsform in entsprechend angepasster Art und Weise für die anderen Ausführungsformen zu verwenden und/oder für Merkmale, die für eine Ausführungsform nicht oder nicht detailliert beschrieben sind, entsprechende Merkmale der anderen Ausführungsformen in angepasster Form anzuwenden.

## Patentansprüche

1. Vorrichtung zur Verankerung eines Fadens (30) in Gewebe, welche Vorrichtung die folgenden Elemente aufweist:
einen am distalen Ende der Vorrichtung angeordneten Anker (1) mit einem Ankerfuss (20) und einer auf einer Schulter des Ankerfusses (20) aufsitzenden Verankerungshülse (22), die ein durch mechanische Schwingungen verflüssigbares Material aufweist oder aus diesem besteht, **gekennzeichnet durch** eine Führungshülse (3) mit einem distalen Teil (3.1) eines kleineren Querschnitts und einem proximalen Teil (3.2) eines grösseren Querschnitts und mit einem axialen Lumen,
eine sich im Lumen der Führungshülse (3) erstreckende Sonotrode (2) mit einem distalen Ende und einem proximalen Ende, wobei das proximale Ende ausgerüstet ist für eine Kopplung der Sonotrode (2) an eine Schwingungsquelle,
den Faden (30), der **durch** den Ankerfuss (20) verläuft,
wobei die Verankerungshülse (22) zwischen dem Ankerfuss (20) und einer Stosshülse (4) oder der Sonotrode (2) oder der Führungshülse (3) eingespannt oder einspannbar angeordnet ist.

2. Vorrichtung nach Anspruch 1, **dadurch gekennzeichnet, dass** ein mittlerer Bereich des Fadens (30) durch den Ankerfuss (20) läuft und zwei Endbereiche des Fadens an einer Aussenseite der Führungshülse (3) befestigt sind.

3. Vorrichtung nach Anspruch 1 oder 2, **dadurch gekennzeichnet, dass** die Verankerungshülse (22) zwischen dem Ankerfuss (20) und der Stosshülse (4) eingespannt ist, wobei der Ankerfuss (20) fest mit der Sonotrode (2) verbunden ist und die Stosshülse (4) Teil einer Stosseinrichtung ist, die zusätzlich eine am proximalen Ende der Stosshülse (4) abgestützte Stossfeder (5) aufweist und die zwischen der Sonotrode (2) und der Führungshülse (3) angeordnet ist.

4. Vorrichtung nach Anspruch 3, **dadurch gekennzeichnet, dass** sie ferner eine zwischen der Stosseinrichtung und der Führungshülse (3) wirkende Spannfeder (7) und einen für eine Entspannung der Stossfeder (5) auf die Spannfeder (7) wirkenden Entspannungshebel (11) aufweist.

5. Vorrichtung nach Anspruch 4, **dadurch gekennzeichnet, dass** die Vorrichtung, wenn die Spannfeder (11) gespannt, die Sonotrode (2) an eine Schwingungsquelle angeschlossen und die Führungshülse (3) an einem Gehäuse, in dem die Schwingungsquelle angeordnet ist, abgestützt ist, einen geschlossenen Lastrahmen darstellt.

6. Vorrichtung nach Anspruch 1 und 2, **dadurch gekennzeichnet, dass** die Verankerungshülse (22) zwischen dem Ankerfuss (20) und der Sonotrode (2) eingespannt ist, wobei die Sonotrode (2) mit der Führungshülse (3) axial verschiebbar wirkverbunden ist und wobei der Ankerfuss (20) durch den Faden (30) mit der Führungshülse (3) verspannt ist.

7. Vorrichtung nach Anspruch 6, **dadurch gekennzeichnet, dass** zwischen der Sonotrode (2) und der Führungshülse (3) eine Feder angeordnet ist, durch die die Sonotrode (2) gegen das distale Ende der Führungshülse (3) gedrückt wird.

8. Vorrichtung nach Anspruch 1 oder 2, **dadurch gekennzeichnet, dass** die Verankerungshülse zwischen dem Ankerfuss (20) und der Führungshülse (3) eingespannt ist, wobei der Ankerfuss (20) am distalen Ende der Sonotrode (2) befestigt ist.

9. Vorrichtung nach Anspruch 8, **dadurch gekennzeichnet, dass** sie ferner eine zwischen der Sonotrode (2) und der Führungshülse (3) wirkende Spannfeder (7) aufweist, durch die die Sonotrode (2) gegen das proximale Ende der Führungshülse (3) gedrückt wird.

10. Vorrichtung nach einem der Ansprüche 3 bis 9, **dadurch gekennzeichnet, dass** der Faden durch eine quer zu einer Achse des Ankerfusses (20) oder durch zwei parallel zur Achse des Ankerfusses (20) verlaufende Öffnungen verläuft.

11. Vorrichtung nach einem der Ansprüche 3 bis 10, **dadurch gekennzeichnet, dass** der Ankerfuss (20) einen Schaftteil (20.1) und einen distal am Schaftteil (20.1) befestigten oder befestigbaren Fussteil (20.2) aufweist.

12. Vorrichtung nach einem der Ansprüche 3 bis 11, **dadurch gekennzeichnet, dass** der Schaftteil (20.1) im wensentlichen rohrförmig ist und dass der Faden (30) in axialer Richtung durch die Schaftteil (20.1) verläuft.

13. Vorrichtung nach einem der Ansprüche 3 bis 12, **dadurch gekennzeichnet, dass** der Faden (30) vom Ankerfuss (20) durch Öffnungen (31 und 32) in der Stosshülse (4) oder der Sonotrode (2) und in der Führungshülse (3) an eine Aussenseite der Führungshülse (3) verläuft.

## Claims

1. A device for the anchoring of a suture (30) in tissue, which device comprises an anchor (1) being arranged at a distal end of the device, the anchor comprising an anchor foot (20) and an anchoring sleeve (22), the anchoring sleeve being positioned on a shoulder of the anchor foot (20) and consisting of, or comprising a material, which is liquefiable by mechanical vibrations,
**characterized by** a guide sleeve (3) with a distal part (3.1) of a smaller cross section, and a proximal part (3.2) of a larger cross section, and with an axial lumen,
a sonotrode (2), which extends in the lumen of the guide sleeve (3), the sonotrode comprising a distal end and a proximal end, wherein the proximal end is designed for a coupling of the sonotrode (2) to a vibration source,
and the suture (30), which runs through the anchor foot (20),
wherein the anchoring sleeve (22) is clamped or clampably arranged between the anchor foot (20) and a pushing sleeve (4), or the sonotrode (2), or the guide sleeve (3).

2. The device according to claim 1, wherein a middle portion of the suture (30) runs through the anchor foot and two end portions of the suture are fastened on an exterior side of the guide sleeve (3).

3. The device according to any one of claims 1 or 2, wherein the anchoring sleeve (22) is clamped between the anchor foot (20) and the pushing sleeve (4), wherein the anchor foot (20) is firmly connected to the sonotrode (2), and wherein the pushing sleeve (4) is a part of a pushing mechanism, which further comprises a pushing spring (5) bearing against a proximal end of the pushing sleeve (4), and which is arranged between the sonotrode (2) and the guide sleeve (3).

4. The device according to claim 3, wherein it further comprises a tension spring (7) and a tension release arm (11), the tension spring (7) being arranged to act between the pushing mechanism and the guide sleeve (3), and the tension release arm (11) being arranged for acting on the tension spring (7) for releasing the tension of the pushing spring (5).

5. The device according to claim 4, wherein it constitutes a closed load frame when the tension spring (7) is tensioned, the sonotrode (2) is coupled to a vibration source, and the guide sleeve (3) is braced against a housing in which the vibration source is located.

6. The device according to claims 1 and 2, wherein the anchoring sleeve (22) is clamped between the anchor foot (20) and the sonotrode (2), wherein the sonotrode (2) is functionally connected to the guide sleeve (3) such that they are capable of axial movement relative to each other, and wherein the anchor foot (20) is biased against the guide sleeve (3) by the suture (30).

7. The device according to claim 6, wherein a spring is situated between the sonotrode (2) and the guide sleeve (3), by means of which the sonotrode (2) is pressed towards the distal end of the guide sleeve (3).

8. The device according to any one of claims 1 or 2, wherein the anchoring sleeve is clamped between the anchor foot (20) and the guide sleeve (3), and wherein the anchor foot (20) is attached to the distal end of the sonotrode (2).

9. The device according to claim 8, wherein it further comprises a tension spring (7) acting between the sonotrode (2) and the guide sleeve (3), by which means the sonotrode (2) is biased towards the proximal end of the guide sleeve (3).

10. The device according to any one of the claims 3 to 9, wherein the suture runs through an opening perpendicular to an axis of the anchor foot (20) or through two openings parallel to the axis of the anchor foot (20).

11. The device according to any one of claims 3 to 10, wherein the anchor foot (20) comprises a shank part (20.1) and a foot part (20.2), and wherein the foot part (20.2) is attached or attachable to the distal end of the shank part (20.1).

12. The device according to any one of claims 3 to 11, wherein the shank part (20.1) is substantially tubular, and wherein the suture runs through the shank part (20.1) in an axial direction.

13. The device according to any one of claims 3 to 12, wherein the suture (30) runs from the anchor foot (20) through openings (31 and 32) in the pushing sleeve (4) or in the sonotrode (2) and along the guide sleeve (3) on an outer side thereof.

## Revendications

1. Dispositif d'ancrage d'un fil (30) dans un tissu, le dispositif présentant un ancrage (1) disposé à une extrémité distale du dispositif et présentant un pied d'ancrage (20) et une douille d'ancrage (22) placée sur un épaulement du pied d'ancrage (20), la douille d'ancrage présentant un matériau apte à être liquéfié sous l'action de vibrations mécaniques ou étant constituée d'un tel matériau,
**caractérisé par**
une douille de guidage (3) dotée d'une partie distale (3.1) de petite section transversale et d'une partie proximale (3.2) de grande section transversale ainsi que d'une lumière axiale,
une sonotrode (2) s'étendant dans la lumière de la douille de guidage (3) et présentant une extrémité distale et une extrémité proximale, l'extrémité proximale étant prévue pour raccorder la sonotrode (2) à une source de vibrations,
le fil (30) qui s'étend par le pied d'ancrage (20) et
la douille d'ancrage (22) serrée ou pouvant être serrée entre le pied d'ancrage (20) et une douille de butée (4) ou la sonotrode (2) ou la douille de guidage (3).

2. Dispositif selon la revendication 1, **caractérisé en ce qu'**une partie centrale du fil (30) traverse le pied d'ancrage (20) et **en ce que** deux parties d'extrémité du fil sont fixées sur un côté extérieur de la douille de guidage (3).

3. Dispositif selon les revendications 1 ou 2, **caractérisé en ce que** la douille d'ancrage (22) est serrée entre le pied d'ancrage (20) et la douille de butée (4), le pied d'ancrage (20) étant relié solidairement à la sonotrode (2) et la douille de butée (4) faisant partie d'un dispositif de butée qui présente de plus un ressort de butée (5) soutenu sur l'extrémité proximale de la douille de butée (4) et étant disposé entre la sonotrode (2) et la douille de guidage (3).

4. Dispositif selon la revendication 3, **caractérisé en ce qu'**il présente en outre un ressort de serrage (7) qui agit entre le dispositif de butée et la douille de guidage (3) et un levier de détente (11) qui agit sur le ressort de serrage (7) pour détendre le ressort de butée (5).

5. Dispositif selon la revendication 4, **caractérisé en ce que** lorsque le ressort de serrage (7) est serré, la sonotrode (2) raccordée à une source de vibrations et la douille de guidage (3) raccordée à un boîtier dans lequel la source de vibrations est disposée, le dispositif constituent un bâti de charge fermé.

6. Dispositif selon les revendications 1 et 2, **caractérisé en ce que** la douille d'ancrage (22) est serrée entre le pied d'ancrage (20) et la sonotrode (2), la sonotrode (2) étant raccordée fonctionnellement à coulissement axial à la douille de guidage (3) et le pied d'ancrage (20) étant serré sur la douille de guidage (3) par le fil (30).

7. Dispositif selon la revendication 6, **caractérisé en ce qu'**un ressort par lequel la sonotrode (2) est repoussée contre l'extrémité distale de la douille de guidage (3) est disposé entre la sonotrode (2) et la douille de guidage (3).

8. Dispositif selon les revendications 1 ou 2, **caractérisé en ce que** la douille d'ancrage est serrée entre le pied d'ancrage (20) et la douille de guidage (3), le pied d'ancrage (20) étant fixé à l'extrémité distale de la sonotrode (2).

9. Dispositif selon la revendication 8, **caractérisé en ce qu'**il présente en outre un ressort de serrage (7) qui agit entre la sonotrode (2) et la douille de guidage (3) et par lequel la sonotrode (2) est repoussée contre l'extrémité proximale de la douille de guidage (3).

10. Dispositif selon l'une des revendications 3 à 9, **caractérisé en ce que** le fil s'étend à travers une ouverture transversale par rapport à un axe du pied d'ancrage (20) ou à travers deux ouvertures s'étendant parallèlement à l'axe du pied d'ancrage (20).

11. Dispositif selon l'une des revendications 3 à 10, **caractérisé en ce que** le pied d'ancrage (20) présente une partie de tige (20.1) et une partie de pied (20.2) fixée ou apte à être fixée distalement sur la partie de tige (20.1).

12. Dispositif selon l'une des revendications 3 à 11, **caractérisé en ce que** la partie de tige (20.1) est essentiellement de forme tubulaire et **en ce que** le fil (30) s'étend dans la partie de tige (20.1) dans la direction axiale.

13. Dispositif selon l'une des revendications 3 à 12, **caractérisé en ce que** le fil (30) s'étend depuis le pied d'ancrage (20) à travers des ouvertures (31 et 32) dans la douille de butée (4) ou la sonotrode (2) et dans la douille de guidage (3) sur le côté extérieur de la douille de guidage (3).
